# EUROPEAN PATENT APPLICATION

(11) **EP 4 400 593 A1**
(43) Date of publication of application: **17.07.2024**
(21) Application number: 23151835.8
(22) Date of filing: 16.01.2023
(51) Int. Cl.: C12N 15/67

(54) **METHODS FOR RNA PRODUCTION**

(71) Applicant: SenseUp GmbH, 52428 Jülich (DE)
(72) Inventor: FLACHBART, Lion, 40789 Monheim a. Rhein (DE); MAHR, Regina, 50226 Frechen (DE); SCHAUMANN, Georg, 50765 Köln (DE)
(74) Representative: Stolmár & Partner Patentanwälte PartG mbB

(57) **Abstract**

The invention relates to a method for producing an RNA of interest, the method comprising a) modifying the sequence of the RNA so that its secondary structure has a lower free energy than before the modification, and b) introducing a DNA encoding the modified RNA of step a) into the cell.

## Description

### Field of the invention

The invention pertains to methods for increasing the production of homo- and heterologous RNA molecules in a cell. Likewise, the invention pertains to cells with increased production of an RNA molecule.

### Technical background

RNA molecules are prone to hydrolysis which hinders *in vivo* production, extraction from cells and storage of homo- and heterologous RNA molecules at high quantity. Different strategies have been used to increase *in vivo* production of recombinant RNA molecules that e.g. incorporate the desired RNA molecule into the secondary structure of tRNA or 5S rRNA scaffold. These methods, however, were not efficient to produce RNA molecules larger than 300 nucleotides at high abundance.

An RNA's secondary structure based on intramolecular base pairing via hydrogen bonds is capable of stabilizing the RNA. In contrast to highly structured tRNAs, rRNAs and sRNAs, mRNA molecules are traditionally less structured which offers the possibility to manage and modulate gene expression in the cell, but likewise renders mRNA molecules accessible to RNAses and hence, more instable.

To predict the secondary structure and thus, the stability of RNA molecules, algorithms have been developed that calculate and draw the thermodynamically most probable RNA secondary structure described by the minimum free energy (ΔG) *in silico,* e.g. LinearDesign or CDSfold. Using these, it is possible to modify the mRNA sequence *in silico* by improving intramolecular base pairing resulting in a thermodynamically optimized and stabilized RNA secondary structure. Due to the redundancy of the triplet code, these algorithms allow for lowering the free energy of the mRNA structure without modifying the resulting amino acid sequence and the protein of interest.

However, it has also been postulated that stabilizing the secondary structure of an mRNA may have a negative effect on protein expression (Terai, G., Kamegai, S., & Asai, K. (2016). CDSfold: an algorithm for designing a protein-coding sequence with the most stable secondary structure. Bioinformatics, 32(6), 828-834. https://doi.org/10.1093/bioinformatics/btv678). So far, however, no evidence exists that *in silico* optimized mRNA secondary structures can be produced with increased stability and lead to an abundance of the respective mRNA in living cells.

### Objective technical problem

It is therefore an aim of the present invention to provide a method for increasing the stability and thus the production of an RNA of interest in a cell.

### Summary of the invention

In one aspect, the invention relates to a method for producing an RNA of interest, the method comprising
a) modifying the sequence of the RNA so that its secondary structure has a lower free energy than before the modification, and
b) introducing a DNA encoding the modified RNA of step a) into the cell.

In another aspect, the invention relates to a method for producing a cell showing an increased abundance of an RNA of interest in a cell, wherein the RNA is capable of forming a secondary structure and encodes an amino acid sequence, the method comprising
a) modifying the sequence of the RNA so that its secondary structure has a lower free energy than before the modification, and
b) introducing a DNA encoding the modified RNA of step a) into the cell.

In another aspect, the invention relates to a method for increasing the abundance of an RNA in a cell, the method comprising: introducing into the cell a DNA encoding the RNA, wherein the RNA is capable of forming a secondary structure, wherein the sequence of the RNA is modified so that its secondary structure has a lower free energy than before the modification.

In another aspect, the invention relates to a method for producing a protein of interest, the method comprising introducing into the cell a DNA encoding a modified RNA, wherein the RNA is capable of forming a secondary structure and encodes the protein of interest, wherein the sequence of the RNA is modified so that its secondary structure has a higher free energy than before the modification while still encoding the protein of interest.

In another aspect, the invention relates to a method for producing a molecule of interest in a cell, the method comprising introducing into the cell a DNA encoding a modified RNA, wherein the RNA is capable of forming a secondary structure and encodes a protein, wherein the sequence of the RNA is modified so that its secondary structure has a higher free energy than before the modification while still encoding the same amino acid sequence as before the modification and wherein the RNA or the protein encoded by the RNA is capable of upregulating the production of the molecule of interest.

In another aspect, the invention relates to a method for producing a molecule of interest in a cell, the method comprising introducing into the cell a DNA encoding a modified RNA, wherein the RNA is capable of forming a secondary structure and encodes an amino acid sequence, wherein the sequence of the RNA is modified so that its secondary structure has a lower free energy than before the modification while still encoding the same amino acid sequence as before the modification and wherein the RNA or the protein encoded by the RNA is capable of downregulating the production of the molecule of interest.

In another aspect, the invention relates to a cell comprising a modified RNA, wherein the RNA is capable of forming a secondary structure, wherein the sequence of the RNA is modified in that its secondary structure has a lower free energy than before the modification.

In another aspect, the invention relates to a pharmaceutical composition comprising an RNA, wherein the RNA is capable of forming a secondary structure and encodes an amino acid sequence, wherein the sequence of the RNA is modified so that its secondary structure has a lower free energy than before the modification while still encoding the same amino acid sequence as before the modification.

### Brief description of the figures

Fig. 1 shows chord diagrams and the MFE ΔG of the secondary structures of *egfp* (wt) and *hRluc* (wt) as well as the thermodynamically optimized RNA sequences *egfp* (opt) and *hRluc* (opt).
Fig. 2 shows the fold induction of broccoli fluorescence shown as ratio of median fluorescence (excitation at 488 nm and emission at 530 nm) and median cell size (FSC) after the addition of DFHBI.
Fig. 3 shows chord diagrams and the MFE ΔG of the secondary structures of *hRluc* (wt) and the destabilized variant *hRluc* (destabilized).
Fig. 4 shows the broccoli fluorescence of DFHBI-stained and unstained cells as ratio of median fluorescence (excitation at 488 nm and emission at 530 nm) and median cell size (FSC).
Fig. 5 shows chord diagrams and the MFE ΔG of the secondary structures of *luc2* (wt) and S *gene* (wt) as well as the thermodynamically optimized RNA sequences *luc2* (opt) and S *gene* (opt).
Fig. 6 shows the fold induction of broccoli fluorescence shown as ratio of median fluorescence (excitation at 488 nm and emission at 530 nm) and median cell size (FSC) after the addition of DFHBI.
Fig. 7 shows the fold induction of eGFP fluorescence shown as ratio of median fluorescence (excitation at 488 nm and emission at 530 nm) and median cell size (FSC) compared to the empty vector control pSU3.
Fig. 8 shows a schema of the biosynthetic pathway for lysine production in *C. glutamicum* highlighting key enzymes aspartokinase (encoded by *lysC*) and homoserine dehydrogenase (encoded by hom).
Fig. 9 shows chord diagrams and the MFE ΔG of the secondary structures of *lysC* (fbr) and *horn* (wt) as well as the thermodynamically destabilized RNA sequence *lysC* (fbr,destabilized) and the thermodynamically stabilized RNA sequence *horn* (stabilized).
Fig. 10 shows chord diagrams and the MFE ΔG of the secondary structures of Ec_*lysC* (fbr) and the thermodynamically destabilized RNA sequence Ec_lysC(fbr,destabilized).

### Detailed description

The term "a RNA/DNA" is herein used synonymously with "a RNA/DNA molecule".

In one aspect, the invention relates to a method for producing an RNA of interest, the method comprising
a) modifying the sequence of the RNA so that its secondary structure has a lower free energy than before the modification, and
b) introducing a DNA encoding the modified RNA of step a) into the cell.

RNA molecules are generally capable of forming a secondary structure. This secondary structure can be modified by exchanging individual bases within the sequence of the RNA, so that intra-molecular base pairing is enhanced and single-stranded regions within the secondary structure are reduced. This leads to a more stable secondary structure characterized by a lower free energy.

The present inventors have surprisingly found that by reducing the minimal free energy of an RNA molecule, the RNA becomes less susceptible to RNases and hydrolysis *in vivo,* in particular in prokaryotic cells. This leads to an accumulation of the modified RNA and increases abundance within the cell, so that yield of the RNA of interest is increased.

The methods according to the invention can be applied to any RNA of interest. In a preferred embodiment, the RNA of interest encodes an amino acid sequence, preferably a protein. In another preferred embodiment, the RNA is an mRNA. Because of the degeneracy of the triplet code, in case of an RNA encoding an amino acid sequence, the modification of the secondary structure can be performed in such a way that after the modification, the RNA still encodes the same amino acid sequence as before the modification. In other words, in a preferred embodiment, the RNA of interest encodes an amino acid sequence and is modified so that its secondary structure has a lower free energy than before the modification while still encoding the same amino acid sequence as before the modification.

In another embodiment, the RNA of interest is a functional RNA. The term "functional RNA" is herein defined as a non-coding RNA that is not translated into a peptide or protein, but fulfils its function in the form of an RNA molecule. For example, the RNA of interest may be a tRNA, an rRNA, a miRNA, an siRNA, a piRNA, a catalytic RNA or an aptamer. In case the RNA of interest is a functional RNA, the modification of the secondary structure is performed so that the RNA can still fulfill the same function as before the modification.

The RNA may be homologous or heterologous with respect to the cell used in the method. In the methods for producing an RNA of interest, the RNA is preferably heterologous to the cell.

Several methods for determining the minimal free energy (MFE) of an RNA molecule and modifying sequence in a way that the thermodynamic stability (and consequently the MFE) of the RNA molecule is increased or decreased have been described in the art (Zhang, H., Zhang, L., Lin, A., Xu, C., Li, Z., Liu, K., Liu, B., Ma, X., Zhao, F., Yao, W., Li, H., Mathews, D. H., Zhang, Y., & Huang, L. (2020). Algorithm for Optimized mRNA Design Improves Stability and Immunogenicity. http://arxiv.org/abs/2004.10177 Terai, G., Kamegai, S., & Asai, K. (2016). CDSfold: an algorithm for designing a protein-coding sequence with the most stable secondary structure. Bioinformatics, 32(6), 828-834. https://doi.org/10.1093/bioinformatics/btv678). The person skilled in the art is therefore able to determine, compare and modify the free energy of different RNA sequences.

The method of the invention comprises a step of introducing a DNA encoding the modified RNA into the cell. The DNA can be introduced into the cell by any method known in the art, e.g., via transformation, transfection or transduction. In a preferred embodiment, the DNA is introduced via electroporation. Once the DNA has been introduced into the cell, it may insert into the genome of the cell, e.g. via homologous recombination or using the CRISPR/Cas system, or remain there as an extrachromosomal DNA molecule, e.g., as a plasmid.

In a preferred embodiment, the method for producing an RNA according to the invention further comprises the steps of
- initiating transcription of the modified RNA; and/or
- extracting the modified RNA from the cell.

The step of initiating expression of the modified RNA may be performed according to any method known to the skilled person. In one embodiment, transcription of the modified RNA is initiated by initiating expression of an RNA polymerase. The transcription of the modified RNA may be performed by the native RNA polymerase or by a heterologous RNA polymerase.

In another embodiment, the RNA of interest is constitutively expressed by the cell, so that transcription does not need to be initiated. Instead, the modified RNA can be directly extracted or harvested from the cell after cultivation for an appropriate amount of time.

The step of extracting the modified RNA from the cell may be performed according to any method known to the skilled person, with or without prior initiation of the transcription of the modified RNA. In some embodiments, it is not necessary to extract the RNA after transcription. Instead, the cell containing the RNA can be directly used for further applications. Both live and dead cells can be used for further applications.

The methods for producing an RNA according to the invention offer the advantage that the abundance of the RNA of interest is increased in the cell compared to the same method in which the non-modified RNA is used. In other words, more RNA of interest can be obtained within the same time unit compared to the same method in which the non-modified RNA is used, so that the amount of the RNA produced is increased compared to the same method in which the RNA without the modification is used.

In another aspect, the invention is directed to a method for producing a cell showing an increased abundance of an RNA of interest in a cell, the method comprising
a) modifying the sequence of the RNA so that its secondary structure has a lower free energy than before the modification, and
b) introducing a DNA encoding the modified RNA of step a) into the cell.

In a preferred embodiment, the RNA of interest encodes an amino acid and is modified so that its secondary structure has a lower free energy than before the modification while still encoding the same amino acid sequence as before the modification.

The invention is further directed to a method for increasing the abundance of an RNA in a cell, the method comprising: introducing into the cell a DNA encoding the RNA, wherein the sequence of the RNA is modified so that its secondary structure has a lower free energy than before the modification while still encoding the same amino acid sequence as before the modification. In a preferred embodiment, the RNA encodes an amino acid and is modified so that its secondary structure has a lower free energy than before the modification while still encoding the same amino acid sequence as before the modification.

In another aspect, the invention relates to a method for producing a protein of interest, the method comprising introducing into the cell a DNA encoding a modified RNA, wherein the RNA is capable of forming a secondary structure and encodes the protein of interest and wherein the sequence of the RNA is modified so that its secondary structure has a higher free energy than before the modification while still encoding the protein of interest. In other words, the method for producing a protein of interest comprises the steps of
a) modifying the sequence of the RNA so that its secondary structure has a higher free energy than before the modification while still encoding the same amino acid sequence as before the modification,
b) introducing a DNA encoding the modified RNA of step a) into the cell, and
c) expressing the protein of interest.

Using the method of the invention, more protein of interest is produced compared to the same method in which the RNA without modification is used for expression of the protein of interest.

In another aspect, the invention is directed to use of a modified RNA for increasing the production of a protein in a cell, the use comprising
a) modifying the sequence of the RNA so that its secondary structure has a higher free energy than before the modification while still encoding the same amino acid sequence as before the modification,
b) introducing a DNA encoding the modified RNA of step a) into the cell, and
c) expressing the protein of interest.

The invention further relates to methods for producing a molecule of interest in a cell. The molecule of interest may be any molecule that can be produced via biofermentation. In a preferred embodiment, the molecule of interest is a primary or a secondary metabolite such as an amino acid, an organic acid, a short chain alcohol, a phenolic acid, a natural metabolite as, e.g., a plant or fungi secondary metabolite or a synthetic metabolite. For example, the molecule to be produced by the method according to the invention is lysine, glutamic acid, isobutanol, succinate, cadaverine, adipic acid or the plant-derived polyphenolic compound resveratrol or piceatannol.

In one embodiment, the method comprises a step of introducing into the cell a DNA encoding a modified RNA, wherein the RNA is capable of forming a secondary structure and encodes a protein, wherein the sequence of the RNA is modified so that its secondary structure has a higher free energy than before the modification while still encoding the same amino acid sequence as before the modification and wherein the RNA or the protein encoded by the RNA is capable of upregulating the production of the molecule of interest.

Herein, the term "upregulating" is understood to relate to any activity of the RNA or the protein encoded by the RNA that directly or indirectly results in an increase in the production of the molecule of interest. In a preferred embodiment, it is the protein encoded by the RNA that is capable of upregulating the production of the molecule of interest. For example, the protein may be an enzyme or a protein that is involved in the production of the molecule within the cell. The protein may also be a transcription factor that upregulates the production of the molecule itself or the expression of enzymes or proteins involved in the production of the molecule.

Without wanting to be bound by a mechanism, it is believed that by rendering the RNA more unstable by increasing the free energy of its secondary structure, the rate of translation into the respective protein is accelerated. This results in the production of more protein. In case said protein upregulates the production of the molecule of interest, an increase in protein will therefore result in an increase in the molecule of interest.

In another embodiment, the method comprises introducing into the cell a DNA encoding a modified RNA, the RNA a) having a sequence capable of forming a secondary structure and b) encoding an amino acid sequence, wherein the sequence of the RNA is modified so that its secondary structure has a lower free energy than before the modification while still encoding the same amino acid sequence as before the modification, said modified RNA or the protein encoded by said modified RNA downregulating the production of the molecule of interest.

Herein, the term "downregulating" is understood to relate to any activity of the RNA or the protein encoded by the RNA that directly or indirectly results in a decrease in the production of the molecule of interest. In a preferred embodiment, it is the protein encoded by the RNA that is capable of downregulating the production of the molecule of interest. For example, the protein may be an enzyme or a protein that hinders the production of the molecule within the cell, e.g. by rerouting the metabolic flux towards the formation of byproducts. The protein may also be a transcription factor that downregulates the production of the molecule itself or the expression of enzymes or proteins involved in the production of the molecule.

By rendering the RNA more stable by decreasing the free energy of its secondary structure, the rate of translation into the respective protein is hindered. This results in the production of less protein. In case said protein downregulates the production of the molecule of interest, a decrease in protein will result in an increase in the molecule of interest.

In the methods for the production of a molecule in a cell, the modified RNA is preferably homologous with respect to the cell used for the production of the molecule. More preferably, the DNA encoding the modified RNA is introduced into the cell in a way that allows the DNA encoding the modified RNA to replace the corresponding endogenous DNA sequence encoding the non-modified RNA within the genome of the cell, so that only the modified RNA is expressed by the cell.

In the methods for the production of a molecule in a cell, the amount of the molecule of interest that is produced is increased compared to the same method in which the RNA without the modification is used.

In the methods for the production of a molecule in a cell, several different RNAs that have different impacts on the production of the molecule may be modified at the same time.

In all methods according to the invention, the cell is preferably a prokaryotic cell, more preferably a bacterial cell, even more preferably a cell from the genus *Corynebacterium* or *Escherichia.* In some embodiments, the cell is a *Pseudomonas syringae* cell, a *Chlamydomonas reinhardtii* cell, an *L. plantarum* cell, a *Yarrowia lipolytica* cell, a S. cerevisiae cell, a *Pichia pastoris* cell, a *Rhodococcus rhodnii* cell, a *C. glutamicum* or *E*. *coli* cell. In the most preferred embodiments, the cell is a *C. glutamicum* or *E*. *coli* cell.

In yet another aspect, the invention is directed to a cell comprising a modified RNA, wherein the RNA is capable of forming a secondary structure and encodes an amino acid sequence, wherein the sequence of the RNA is modified in that its secondary structure has a lower free energy than before the modification.

The modified RNA may be homologous or heterologous to the cell of the invention.

The cell according to the invention is preferably a bacterial cell, more preferably a cell from the genus *Corynebacterium* or *Escherichia.* In the most preferred embodiments, the cell is a *C. glutamicum* or *E*. *coli* cell.

In another aspect, the invention relates to pharmaceutical compositions comprising an RNA, wherein the RNA is capable of forming a secondary structure and encodes an amino acid sequence, wherein the sequence of the RNA is modified so that its secondary structure has a lower free energy than before the modification while still encoding the same amino acid sequence as before the modification.

### Examples

### Example 1

Examination of thermodynamically optimized RNA secondary structures of *egfp* and *hRluc* produced *in vivo* in comparison to their corresponding wildtype sequences in *C*. *glutamicum*

### a) Calculating thermodynamically stabilized RNA secondary structures of egfp and hRluc

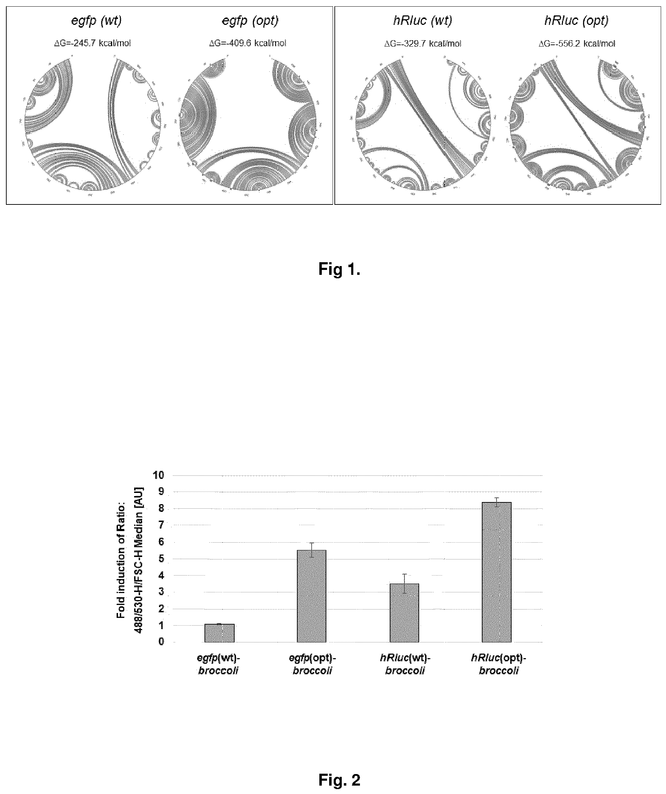

The coding sequences (CDS) of the enhanced green fluorescent protein encoding sequence *egfp* (SEQ ID No.1; originally adapted from the jellyfish *Aequorea victoria*) and the luciferase encoding sequence *hRluc* (SEQ ID No.3; originally adapted from *Renilla reniformis*) were analyzed by LinearFold to calculate the minimum free energy (MFE) ΔG of their corresponding RNA sequences and to draw the corresponding RNA secondary structure, respectively (Figure 1) (Huang, L., Zhang, H., Deng, D., Zhao, K., Liu, K., Hendrix, D. A., & Mathews, D. H. (2019). LinearFold: Linear-time approximate RNA folding by 5'-to-3' dynamic programming and beam search. Bioinformatics, 35(14), i295-i304. https://doi.org/10.1093/bioinformatics/btz375). By adjusting a beam size of 50, the calculated MFE for *egfp* was ΔG=-245.7 kcal/mol and the MFE for *hRluc* was ΔG=-329.7 kcal/mol.

Subsequently, the CDS of *egfp* (wt) and *hRluc* (wt) were optimized by the LinearDesign algorithm in a way that the thermodynamic stability of the corresponding RNA sequences was increased resulting in a reduced MFE of -409.6 kcal/mol for *egfp* (opt) (SEQ ID No.2) and -556.2 kcal/mol for *hRluc* (opt) (SEQ ID No.4) (Figure 1) (Zhang, H., Zhang, L., Lin, A., Xu, C., Li, Z., Liu, K., Liu, B., Ma, X., Zhao, F., Yao, W., Li, H., Mathews, D. H., Zhang, Y., & Huang, L. (2020). Algorithm for Optimized mRNA Design Improves Stability and Immunogenicity. http://arxiv.org/abs/2004.10177). The algorithm calculates an RNA molecule sequence with improved thermodynamic stability altering the codon usage, but without modifying the final protein sequence.

### b) Construction of the vectors encoding the wildtype and optimized CDS of egfp and hRluc

The construction of the plasmid vector was achieved by means of chemical synthesis of synthetic DNA fragments. The DNA fragments include the promoter sequence P_{T7}, a 5'UTR, a CDS, the F30 scaffold with a broccoli aptamer in the first integration point and a "UUCG spacer" in the second integration point, and a terminator sequence T_{BFK20}. As CDS, the wildtype CDS of *egfp* (wt) or *hRluc* (wt), or the modified sequences for *egfp* (opt) or *hRluc* (opt) resulting in thermodynamically stabilized RNA secondary structures were added, respectively (SEQ ID No.5 for P_{T7}-*egfp*(wt)-*broccoli*; SEQ ID No.6 for P_{T7}-*egfp*(*opt*)*-broccoli;* SEQ ID No.7 for P_{T7}-*hRluc*(wt)-*broccoli;* SEQ ID No.8 for P_{T7}-*hRluc*(*opt*)*-broccoli).* The synthetic DNA fragments were incorporated in the high copy vector pSU1 (SEQ ID No.9) resulting in vectors pSU1_P_{T7}*-egfp*(wt)-*broccoli* (SEQ ID No.10), pSU1_P_{T7}-*egfp*(opt)-*broccoli* (SEQ ID No.11), pSU1_P_{T7}-*hRluc*(wt)-*broccoli* (SEQ ID No.12) and pSU1_P_{T7}*-hRluc*(opt)-*broccoli* (SEQ ID No.13).

### c) Transformation of C. glutamicum with pSU1_P_{T7}-egfp(wt)-broccoli, pSU1_P_{T7}-egfp(opt)-broccoli, pSU1_P_{T7}-hRluc(wt)-broccoli and pSU1_P_{T7}-hRluc(opt)-broccoli

Competent cells of *C. glutamicum* strain ATCC13032 (DE3) *Δcg2273* were prepared and transformed with plasmid pSU1_P_{T7}-*egfp*(wt)-*broccoli*, pSU1_P_{T7}-*egfp*(opt)-*broccoli*, pSU1_P_{T7}*-hRluc*(wt)-*broccoli* and pSU1_P_{T7}-*hRluc*(opt)-*broccoli* according to Tauch et al. (Tauch, A., Kirchner, O., L6ffler, B., Götker, S., Pühler, A., & Kalinowski, J. (2002). Efficient electrotransformation of Corynebacterium diphtheriae with a mini-replicon derived from the Corynebacterium glutamicum plasmid pGA1. Current Microbiology, 45(5), 362-367. https://doi.org/10.1007/s00284-002-3728-3). The selection of transformants was carried out on CGIII agar (1 %) plates with 25 µg/ml kanamycin (Menkel, E., Thierbach, G., Eggeling, L., Sahm, H., & Asta Pharma Gruppe, D. A. (1989). Influence of Increased Aspartate Availability on Lysine Formation by a Recombinant Strain of Corynebacterium glutamicum and Utilization of Fumarate. In APPLIED AND ENVIRONMENTAL MICROBIOLOGY (Vol. 55, Issue 3).). Clones thus obtained were named *C. glutamicum* ATCC 13032 (DE3) *Δcg2273* pSU1_P_{T7}-*egfp*(wt)-*broccoli, C. glutamicum* ATCC 13032 (DE3) *Δcg2273* pSU1_P_{T7}-*egfp*(opt)-*broccoli, C. glutamicum* ATCC 13032 (DE3) *Δcg2273* pSU1_P_{T7}-*hRluc*(wt)-*broccoli* or *C. glutamicum* ATCC 13032 (DE3) *Δcg2273* pSU1_P_{T7}-*hRluc*(opt)-*broccoli,* depending on which plasmid was used for transformation.

### d) Cultivation and validation of cells using fluorescent activated cell sorting (FACS)

The produced strains *C. glutamicum* ATCC 13032 (DE3) *Δcg2273* pSU1_P_{T7}-*egfp*(wt)-*broccoli, C. glutamicum* ATCC 13032 (DE3) *Δcg2273* pSU1_P_{T7}-*egfp*(opt)-*broccoli*, *C. glutamicum* ATCC 13032 (DE3) *Δcg2273* pSU1_P_{T7}-*hRluc*(wt)-*broccoli* or *C. glutamicum* ATCC 13032 (DE3) *Δcg2273* pSU1_P_{T7}-*hRluc*(opt)-*broccoli* were each streaked on a BHI agar plate containing a final concentration of 25 µg/ml kanamycin, which were cultivated at 30°C overnight. Grown cells were resuspended in CGIII medium with 25 µg/mL kanamycin and the OD₆₀₀ was adjusted to 0.75 in a tube containing 2 mL CGIII cultivation medium containing a final concentration of 25 µg/ml kanamycin. Cells were incubated at 30°C and 120 rpm for six hours. Subsequently, the expression of genomically encoded T7 polymerase was induced by adding a final concentration of 1.5 mM IPTG and incubated for further six hours by agitation at 120 rpm and 30°C.

All RNAs under study contain the aptamer *broccoli* transcriptionally fused to their 3' ends. *Broccoli* serves as appropriate readout signal for measuring the abundancy of the *in vivo* produced RNA molecules of interest in the bacterial cell and verifies their full-length production. The *broccoli* aptamer forms an RNA secondary structure that binds the fluorophore DFHBI (3,5-difluoro-4-hydroxybenzylidene imidazolinone) and in turn shows green fluorescence (Filonov, G. S., Moon, J. D., Svensen, N., & Jaffrey, S. R. (2014). Broccoli: Rapid selection of an RNA mimic of green fluorescent protein by fluorescence-based selection and directed evolution. Journal of the American Chemical Society, 136(46), 16299-16308. https://doi.org/10.1021/ja508478x). To measure and verify the production of the RNA under study, grown cells were diluted to an OD₆₀₀ of 0.6 using PBS with a final concentration of 500 µM DFHBI. After 10 min of incubation, the cell suspension was analyzed using an Arialll High-speed cell sorter (BD Biosciences, Franklin Lakes, NJ, USA) equipped with a 70 µm nozzle and run with a sheath pressure of 70 psi. A 488 nm blue solid laser was used for excitation. Forward-scatter characteristics (FSC) were recorded as small-angle scatter and side-scatter characteristics (SSC) were recorded as orthogonal scatter of the 488 nm laser. A 502 nm long-pass and 530/30 nm band-pass filter combination were used for fluorescence detection. FACSDiva 8.0.1 (BD Biosciences, San Jose, USA) was used for FACS control and data analysis. Prior to data acquisition, debris and electronic noise were excluded from the analysis by electronic gating in the FSC-H against SSC-H plot. Another gating step was performed on the resulting population in the FSC-H against FSC-W plot to exclude doublets. Fluorescence acquisition was performed with the population resulting from this two-step gating.

*Egfp*(*wt*)*-broccoli-RNA* producing cells (*C. glutamicum* ATCC 13032 (DE3) Δ*cg2273* pSU1_P_{T7}-*egfp*(wt)-*broccoli*) did not show any increase in broccoli fluorescent activity upon addition of DFHBI demonstrating that no *egfp*(*wt*)*-broccali-RNA* was formed in the bacterial cells (Figure 2). In contrast, the *egfp*(opt)*-brocco*/*i*-RNA with optimized RNA secondary structure (*C. glutamicum* ATCC 13032 (DE3) *Δcg2273* pSU1_P_{T7}-*egfp*(opt)-*broccoli*) revealed a 5.5-fold increased ratio of median fluorescence to cell size demonstrating that the optimized RNA variant was stably produced in the bacterial cell (Figure 2). The *hRluc*(*wt*)*-broccali*-RNA produced by strain *C. glutamicum* ATCC 13032 (DE3) *Δcg2273* pSU1_P_{T7}-*hRluc*(wt)-*broccoli* revealed a 3.5-fold increase ratio of median fluorescence to cell size upon DFHBI addition, while the ratio of median fluorescence to cell size of the thermodynamically optimized *hRluc*(opt)*-broccoli*-RNA was 8.4-fold increased in strain *C. glutamicum* ATCC 13032 (DE3) *Δcg2273* pSU1_P_{T7}-*hRluc*(opt)-*broccoli* (Figure 2). The results show that the abundance of RNA with improved thermodynamic stability was significantly increased compared to the wildtype sequences.

### Example 2

### Destabilization of RNA by reducing the thermodynamic stability

### a) Calculating a thermodynamically instabile RNA secondary structure of hRluc

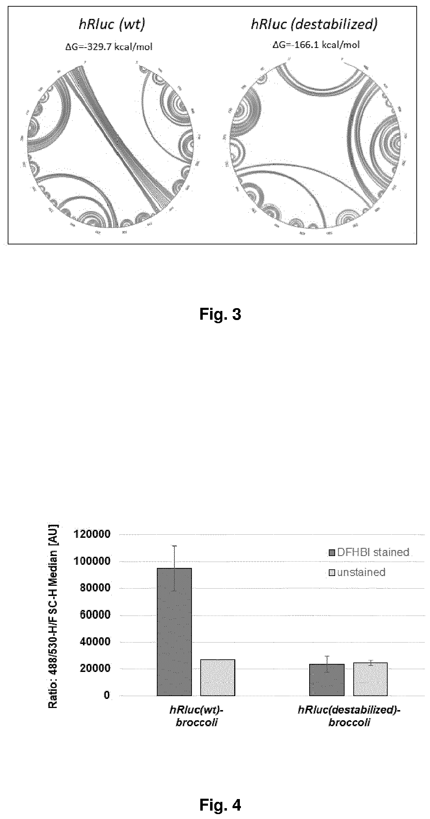

The coding sequence (CDS) of the luciferase encoding sequence *hRluc* (SEQ ID No.3; originally adapted from *Renilla reniformis*) was analyzed by LinearFold to calculate the minimum free energy (MFE) ΔG of its corresponding RNA sequence and to draw the corresponding RNA secondary structure, respectively (Huang, L., Zhang, H., Deng, D., Zhao, K., Liu, K., Hendrix, D. A., & Mathews, D. H. (2019). LinearFold: Linear-time approximate RNA folding by 5'-to-3' dynamic programming and beam search. Bioinformatics, 35(14), i295-i304. https://doi.org/10.1093/bioinformatics/btz375). The calculated MFE for *hRluc* (wt) was ΔG=-333.1 kcal/mol. Subsequently, the codon-usage of the CDS of *hRluc* (wt) was modified to yield the CDS *hRluc* (destabilized) (SEQ ID No.14), in a way that the resulting RNA sequence featured reduced thermodynamic stability with a MFE of -160.1 kcal/mol (Figure 3).

### b) Construction of the vector pSU1_P_{T7}-hRluc(destabilized)-broccoli

Construction of the plasmid vector was achieved by means of chemical synthesis of a synthetic DNA fragment. The DNA fragment includes the promoter sequence P_{T7}, a 5'UTR, the CDS of *hRluc* (destabilized), the F30 scaffold with a broccoli aptamer in the first integration point and a "UUCG spacer" in the second integration point, and a terminator sequence T_{BFK20} (SEQ ID No.15). The synthetic DNA fragment was incorporated into the high copy vector pSU1 (SEQ ID No.9) resulting in vector pSU1_P_{T7}-*hRluc(destabilized)-broccoli* (SEQ ID No.16).

### c) Transformation of C. glutamicum ATCC13032 (DE3) Δcg2273 with plasmid pSU1_P_{T7}-hRluc(destabilized)-broccoli

Competent cells of *C. glutamicum* strain ATCC 13032 (DE3) *Δcg2273* were prepared and transformed with plasmid pSU1_P_{T7}-*hRluc*(destabilized)-*broccoli* according to Tauch et al. (Tauch, A., Kirchner, O., L6ffler, B., Götker, S., Pühler, A., & Kalinowski, J. (2002). Efficient electrotransformation of Corynebacterium diphtheriae with a mini-replicon derived from the Corynebacterium glutamicum plasmid pGA1. Current Microbiology, 45(5), 362-367. https://doi.org/10.1007/s00284-002-3728-3). The selection of the transformants was carried out on CGIII agar (1 %) plates with 25 µg/ml of kanamycin (Menkel, E., Thierbach, G., Eggeling, L., Sahm, H., & Asta Pharma Gruppe, D. A. (1989). Influence of Increased Aspartate Availability on Lysine Formation by a Recombinant Strain of Corynebacterium glutamicum and Utilization of Fumarate. In APPLIED AND ENVIRONMENTAL MICROBIOLOGY (Vol. 55, Issue 3).). Clones thus obtained were named *C. glutamicum* ATCC 13032 (DE3) Δ*cg2273* pSU1_P_{T7}-*hRluc*(destabilized)-*broccoli*.

### d) Cultivation and validation of cells using fluorescent activated cell sorting (FACS)

The strains *C. glutamicum* ATCC 13032 (DE3) *Δcg2273* pSU1_P_{T7}*-hRluc*(wt)-*broccoli* (example 1) and *C. glutamicum* ATCC 13032 (DE3) *Δcg2273* pSU1_P_{T7}-*hRluc*(destabilized)-*broccoli* were each streaked on BHI agar plates containing a final concentration of 25 µg/ml kanamycin, which were cultivated at 30°C overnight. Grown cells were resuspended in CGIII medium and the OD₆₀₀ was adjusted to 0.75 in a tube containing 2 mL CGIII cultivation medium containing a final concentration of 25 µg/ml kanamycin. Cells were incubated at 30°C and 120 rpm for six hours. Subsequently, the expression of genomically encoded T7 polymerase was induced by adding a final concentration of 1.5 mM IPTG and incubated for further six hours by agitation at 120 rpm and 30°C. Then, cells were diluted to an OD₆₀₀ of 0.6 using PBS with a final concentration of 500 µM DFHBI. After 10 min of incubation, the cell suspension was analyzed using an Arialll High-speed cell sorter (BD Biosciences, Franklin Lakes, NJ, USA) as already described in example 1.

Cells producing the *hRluc*(wt)*-broccoli*-RNA revealed a 3.5-fold increase in fluorescent activity upon DFHBI addition (Figure 4). In contrast, the ratio of broccoli fluorescent signal to cell size of DFHBI-stained *C. glutamicum* ATCC 13032 (DE3) *Δcg2273* pSU1_P_{T7}-*hRluc*(destabilized)-*broccoli* cells was not increased compared to the unstained control cells. The results show that the destabilization of the *hRluc* RNA secondary structure resulted in a strong reduction of the *hRluc*(destabilized)-*broccoli* RNA abundance in the bacterial cell.

### Example 3

*In vivo* production of the thermodynamically stabilized RNA secondary structure of *egfp* (opt) in comparison to the wildtype sequence *egfp* (wt) in *E. coli*

### a) Calculating thermodynamically stabilized RNA secondary structures of egfp

The CDS of *egfp* (wt) (SEQ ID No.1) was optimized according to the procedure described in example 1a resulting in *egfp* (opt) (SEQ ID No.2).

### b) Construction of the vectors encoding the wildtype and RNA secondary structure optimized CDS of egfp

The construction of the plasmid vector was achieved by means of chemical synthesis of synthetic DNA fragments. The DNA fragments include the promoter sequence P_{T7}, a 5'UTR, the wildtype or the optimized CDS of *egfp,* the F30 scaffold with a broccoli aptamer in the first integration point and a "UUCG spacer" in the second integration point, and a terminator sequence T_{BFK20} (SEQ ID No.5 for P_{T7}-*egfp*(wt)-*broccoli* and SEQ ID No.6 for P_{T7}-*egfp*(opt)-*broccoli*)*.* Each of the synthetic DNA fragments was incorporated into the high copy *C. glutamicum-E. coli* shuttle vector pSU1 (SEQ ID No.9) resulting in vectors pSU1_P_{T7}-*egfp*(wt)-*broccoli* (SEQ ID No.10) and pSU1_P_{T7}-*egfp*(opt)-*broccoli* (SEQ ID No.11) as already described in example 1b.

### c) Transformation of E. coli with vector pSU1_P_{T7}-egfp(wt)-broccoli or vector pSU1_P_{T7}-egfp(opt)-broccoli

For transformation of plasmid vectors in *E. coli* BL21(DE3) cells, the transformation and storage solution (TSS) transformation protocol according to Chung et al., 1989 was used (Chung, C. T., Niemela, S. L., & Miller, R. H. (1989). One-step preparation of competent Escherichia coli: transformation and storage of bacterial cells in the same solution. Proceedings of the National Academy of Sciences of the United States of America, 86(7), 2172-2175. https://doi.org/10.1073/pnas.86.7.2172). A single colony of the target strain was inoculated in a tube containing 3 mL LB medium and grown until an OD₆₀₀ between 0.3 and 0.8 was reached. Subsequently, the culture was chilled on ice for ten minutes. An equal volume (3 mL) of ice cold 2× TSS (8 g/L Bacto-Tryptone, 5 g/L Yeast Extract, 5 g/L NaCl, 200 g/L PEG 8000) was added and the tube was vortexed thoroughly by avoiding warming up the cells. The bacterial suspension was incubated for further ten minutes on ice. To 1 mL of competent cells at least 10 ng plasmid DNA were added and mixed by vortexing. The suspension was then left on ice for 30 minutes and 200 µL of the culture were plated on LB agar plates (1%) containing 50 µg/mL kanamycin. Resulting strains were named *E. coli* BL21(DE3)_ pSU1_P_{T7}-*egfp*(wt)-*broccoli* and *E. coli* BL21 (DE3)_ pSU1_P_{T7}*egfp*(opt)-*broccoli.*

### d) Cultivation and phenotypic validation of cells using fluorescent activated cell sorting (FACS)

The *E. coli* BL21 (DE3)_ pSU1_P_{T7}-*egfp*(wt)-*broccoli* and *E*. *coli* BL21 (DE3)_ pSU1_P_{T7}-*egfp*(opt)-*broccoli* cells were inoculated from a single colony in a tube containing 2 mL 2x YT medium (16g/L tryptone, 10g yeast extract, 5g NaCl, pH adjusted to 5 with NaOH) (Sambrook, J., Fritsch, E.F., T, M., 1989. Molecular Cloning: A Laboratory Manual. Cold Spring Harbor Laboratory Press, Cold Spring Harbor, New York.) with 50 µg/mL kanamycin and cultivated overnight at 37°C and 120 rpm. The next day, the pre-culture was used to inoculate 2 mL fresh 2x YT medium containing 50 µg/mL kanamycin to an OD₆₀₀ of 0.1. Cells were grown at 37°C and 120 rpm and after three hours, expression of T7 RNA polymerase was induced by addition of 0.4 mM IPTG. Cultivation was continued for further four hours. Then, cells were diluted to an OD₆₀₀ of 0.6 using PBS with a final concentration of 50 µM DFHBI. After 10 min of incubation, the cell suspension was analyzed by FACS as already described in example 1d. *Egfp*(wt)-*broccoli*-RNA producing *E. coli* BL21(DE3) cells did not show any increase in *broccoli* fluorescent activity upon addition of DFHBI demonstrating that no full-length *egfp*(wt)*-broccali*-RNA is present in the cells. In contrast, the *egfp*(opt)*-broccoli*-RNA with optimized RNA secondary structure revealed a significantly increased fluorescent signal demonstrating that the optimized RNA variant was stably produced in the bacterial cell.

### Example 4

RNA secondary structure optimization of *luc2* and S *gene* encoding SARS-CoV2-spike-protein and *in vivo* production of the corresponding RNA in *C. glutamicum*

### a) Calculating thermodynamically stabilized RNA secondary structures of luc2 mRNA and S gene encoding SARS-CoV2-spike-protein

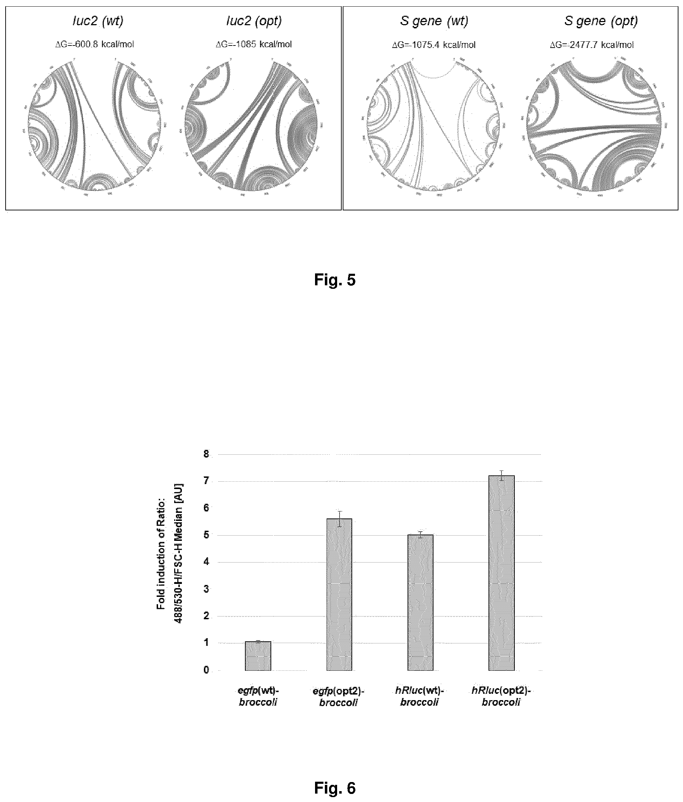

A modified CDS sequence with increased thermodynamic stability was determined for *luc2* encoding firefly luciferase deriving from *Photinus pyralis* (SEQ ID No.17). While the original sequence of luc2(wt) featured a MFE of ΔG=-600.8 kcal/mol, the modified sequence luc2(opt) resulted in an RNA with increased stability of its secondary structure (SEQ ID No.18; MFE ΔG=-1085 kcal/mol) (Figure 5).

In addition, the DNA sequence encoding SARS-CoV2-spike-protein (surface glycoprotein encoded by *S gene*(wt); SEQ ID No.19; MFE ΔG=-1075.4 kcal/mol) was modified for increased thermodynamic RNA stability (S gene(opt); SEQ ID No.20) resulting in a MFE ΔG=-2477.7 kcal/mol (Figure 5).

### b) Construction of the vectors encoding the wildtype as well as the RNA secondary structure optimized CDS of luc2 and S gene encoding SARS-CoV2-spike-protein

The construction of the plasmid vectors was achieved by means of chemical synthesis of synthetic DNA fragments. The DNA fragments include the promoter sequence P_{T7}, a 5'UTR, a CDS optimized for increased thermodynamic stability of the encoded RNA, the F30 scaffold with a *broccoli* aptamer in the first integration point and a "UUCG spacer" in the second integration point, and a terminator sequence T_{BFK20}. The wildtype or the modified sequences for *luc2* (*luc2*(wt) and luc2(opt)) or SARS-Cov2-spike protein (S *gene*(wt) and S gene(opt)) were encoded in the DNA fragment, respectively (SEQ ID No.21 for *P_{T7}-luc2*(wt)-*broccoli,* SEQ ID No.22 for *P_{T7}-luc2*(opt)-*broccoli,* SEQ ID No.23 for *P_{T7}-S gene(wt)-broccoli,* SEQ ID No.24 for *P_{T7}-S gene*(opt)-*broccoli*). The synthetic DNA fragments were incorporated in the high copy vector pSU1 (SEQ ID No.9), respectively, resulting in plasmid vectors pSU1_P_{T7}*-luc2*(wt)-*broccoli* (SEQ ID No.25), pSU1_P_{T7}-*luc2(opt)-broccoli* (SEQ ID No.26), pSU1_P_{T7}-*S gene*(wt)*-broccoli* (SEQ ID No.27) and pSU1_P_{T7}-S gene(opt)-*broccoli* (SEQ ID No.28).

### c) Transformation of C. glutamicum ATCC13032 (DE3) Δcg2273 with pSU1_P_{T7}-luc2(wt)-broccoli, pSU1_P_{T7}-luc2(opt)-broccoli, pSU1_P_{T7}-S gene(wt)-broccoli and pSU1_P_{T7}-S gene(opt)-broccoli

Competent cells of *C. glutamicum* strain ATCC13032 (DE3) *Δcg2273* were prepared and transformed with plasmid vectors pSU1_P_{T7}-*luc2*(wt)-*broccoli*, pSU1_P_{T7}-*luc2*(opt)-*broccoli,* pSU1_P_{T7}-*S gene*(wt)-*broccoli* and pSU1_P_{T7}-*S gene(opt)-broccoli* according to Tauch et al. (Tauch, A., Kirchner, O., L6ffler, B., Götker, S., Pühler, A., & Kalinowski, J. (2002). Efficient electrotransformation of Corynebacterium diphtheriae with a mini-replicon derived from the Corynebacterium glutamicum plasmid pGA1. Current Microbiology, 45(5), 362-367. https://doi.org/10.1007/s00284-002-3728-3), respectively. The selection of the transformants was carried out on CGIII agar (1 %) plates with 25 µg/ml of kanamycin (Menkel, E., Thierbach, G., Eggeling, L., Sahm, H., & Asta Pharma Gruppe, D. A. (1989). Influence of Increased Aspartate Availability on Lysine Formation by a Recombinant Strain of Corynebacterium glutamicum and Utilization of Fumarate. In APPLIED AND ENVIRONMENTAL MICROBIOLOGY (Vol. 55, Issue 3).). Clones thus obtained were named *C. glutamicum* ATCC 13032 (DE3) *Δcg2273* pSU1_P_{T7}-*luc2*(wt)-*broccoli, C. glutamicum* ATCC 13032 (DE3) *Δcg2273* pSU1_P_{T7}*-luc2*(opt)-*broccoli, C. glutamicum* ATCC 13032 (DE3) *Δcg2273* pSU1_*P_{T7}-S gene(wt)-broccali* or *C. glutamicum* ATCC 13032 (DE3) *Δcg2273* pSU1_P_{T7}-*S gene(opt)-broccoli,* depending on which plasmid was used for transformation.

### d) Cultivation and phenotypic validation of cells using fluorescent activated cell sorting (FACS)

The produced strains *C. glutamicum* ATCC 13032 (DE3) *Δcg2273* pSU1_P_{T7}-*luc2*(wt)-*broccoli, C. glutamicum* ATCC 13032 (DE3) *Δcg2273* pSU1_P_{T7}*-luc2*(opt)-*broccoli, C. glutamicum* ATCC 13032 (DE3) *Δcg2273* pSU1_*P_{T7}-S gene(wt)-broccoli* or *C. glutamicum* ATCC 13032 (DE3) *Δcg2273* pSU1_P_{T7}-*S gene(opt)-broccoli* were each streaked on BHI agar plates containing a final concentration of 25 µg/ml kanamycin, which were cultivated at 30°C overnight. Grown cells were resuspended in CGIII medium and the OD₆₀₀ was adjusted to 0.75 in a tube containing 2 mL CGIII cultivation medium containing a final concentration of 25 µg/ml kanamycin. Cells were incubated at 30°C and 120 rpm for six hours. Subsequently, the expression of genomically encoded T7 polymerase was induced by adding a final concentration of 1.5 mM IPTG and incubated for further six hours by agitation at 120 rpm and 30°C. Then, cells were diluted to an OD₆₀₀ of 0.6 using PBS with a final concentration of 500 µM DFHBI. After 10 min of incubation, the cell suspension was analyzed using an Arialll High-speed cell sorter (BD Biosciences, Franklin Lakes, NJ, USA).

The fluorescent output of DFHBI-bound *broccoli* was significantly increased for the molecules with stabilized RNA secondary structures *luc2*(opt)*-broccali*-RNA and S *gene*(opt)*-braccoli*-RNA compared to the wildtype constructs *luc2*(wt)*-broccoli*-RNA and S *gene*(wt)*-broccoli*-RNA. The results demonstrate that the thermodynamic stabilization leads to an increased *in vivo* abundance of the RNAs of interest.

### Example 5

### Stabilization of the RNA secondary structure using the CDSfold algorithm

### a) Calculating stable RNA secondary structures of egfp and hRluc using CDSfold

Using the CDSfold algorithm, stable RNA secondary structures of *egfp* and *hRluc* were calculated based on the wildtype sequences of *egfp*(wt) (SEQ ID No.1) and *hRluc*(wt) (SEQ ID No.3) shown in example 1 (Terai, G., Kamegai, S., & Asai, K. (2016). CDSfold: an algorithm for designing a protein-coding sequence with the most stable secondary structure. Bioinformatics, 32(6), 828-834. https://doi.org/10.1093/bioinformatics/btv678). The CDSfold stabilized secondary structure for egfp(opt2) (SEQ ID No.29) featured a MFE of ΔG=-437.2 kcal/mol, whereas a stabilized secondary structure with a MFE of ΔG=-574.3 kcal/mol was calculated for *hRluc*(opt2) (SEQ ID No.30).

### b) Construction of the vectors encoding the CDS of egfp(opt2) and hRluc(opt2)

The construction of the plasmid vector was achieved by means of chemical synthesis of synthetic DNA fragments. The DNA fragments include the promoter sequence P_{T7}, a 5'UTR, a CDS optimized via the CDSfold algorithm, the F30 scaffold with a *broccoli* aptamer in the first integration point and a "UUCG spacer" in the second integration point, and a terminator sequence T_{BFK20}. The CDSfold optimized sequences for *egfp* (egfp(opt2)) or *hRluc (hRluc*(opt2)) were encoded in the DNA fragment, respectively (SEQ ID No.31 for *P_{T7}-egfp*(opt2)-*broccoli* and SEQ ID No.32 for *P_{T7}-hRluc*(opt2)-*broccoli*)*.* The synthetic DNA fragments were incorporated in the high copy vector pSU1 (SEQ ID No.9), respectively, resulting in plasmid vectors pSU1_P_{T7}-*egfp*(opt2)-*broccoli* (SEQ ID No.33) and pSU1_P_{T7}-*hRluc*(opt2)-*broccoli* (SEQ ID No.34).

### c) Transformation of C. glutamicum ATCC13032 (DE3) Δcg2273 with pSU1_P_{T7}-egfp(opt2)-broccoli and pSU1_P_{T7}-hRluc(opt2)-broccoli

Competent cells of *C. glutamicum* strain ATCC13032 (DE3) *Δcg2273* were prepared and transformed with plasmid pSU1_P_{T7}-*egfp*(opt2)-*broccoli* and pSU1_P_{T7}-*hRluc*(opt2)-*broccoli* according to Tauch et al. (Tauch, A., Kirchner, O., L6ffler, B., Götker, S., Pühler, A., & Kalinowski, J. (2002). Efficient electrotransformation of Corynebacterium diphtheriae with a mini-replicon derived from the Corynebacterium glutamicum plasmid pGA1. Current Microbiology, 45(5), 362-367. https://doi.org/10.1007/s00284-002-3728-3). The selection of the transformants was carried out on CGIII agar (1 %) plates with 25 µg/ml of kanamycin (Menkel, E., Thierbach, G., Eggeling, L., Sahm, H., & Asta Pharma Gruppe, D. A. (1989). Influence of Increased Aspartate Availability on Lysine Formation by a Recombinant Strain of Corynebacterium glutamicum and Utilization of Fumarate. In APPLIED AND ENVIRONMENTAL MICROBIOLOGY (Vol. 55, Issue 3).). Clones thus obtained were named *C. glutamicum* ATCC 13032 (DE3) *Δcg2273* pSU1_P_{T7}-*egfp*(opt2)-*broccoli* or *C. glutamicum* ATCC 13032 (DE3) *Δcg2273* pSU1_P_{T7}*-hRluc*(opt2)-*broccoli,* depending on which plasmid was used for transformation.

### d) Cultivation and phenotypic validation of cells using fluorescent activated cell sorting (FACS)

The produced strains *C. glutamicum* ATCC 13032 (DE3) *Δcg2273* pSU1_P_{T7}-*egfp*(opt2)-*broccoli* and *C. glutamicum* ATCC 13032 (DE3) *Δcg2273* pSU1_P_{T7}-*hRluc*(opt2)-*broccoli,* as well as wildtype strains *C. glutamicum* ATCC 13032 (DE3) *Δcg2273* pSU1_P_{T7}-*egfp*(wt)-*broccoli* and *C. glutamicum* ATCC 13032 (DE3) *Δcg2273* pSU1_P_{T7}-*hRluc*(wt)-*broccoli* were each streaked on BHI agar plates containing a final concentration of 25 µg/ml kanamycin, which were cultivated at 30°C overnight. Grown cells were resuspended in CGIII medium and the OD₆₀₀ was adjusted to 0.75 in a tube containing 2 mL CGIII cultivation medium containing a final concentration of 25 µg/ml kanamycin. Cells were incubated at 30°C and 120 rpm for six hours. Subsequently, the expression of genomically encoded T7 polymerase was induced by adding a final concentration of 1.5 mM IPTG and incubated for further six hours by agitation at 120 rpm and 30°C. Then, cells were diluted to an OD₆₀₀ of 0.6 using PBS with a final concentration of 500 µM DFHBI. After 10 min of incubation, the cell suspension was analyzed using an Arialll High-speed cell sorter (BD Biosciences, Franklin Lakes, NJ, USA).

*Egfp*(wt)*-broccoli*-RNA producing cells did not show any increase in *broccoli* fluorescent activity upon addition of DFHBI demonstrating that no *egfp*(wt)*-broccoli*-RNA is formed in the bacterial cells (Figure 6). In contrast, the *egfp*(opt2)*-broccoli*-RNA with optimized RNA secondary structure revealed a 5.6-fold increased ratio of median fluorescence to cell size demonstrating that the optimized RNA variant was more stably produced in the bacterial cell (Figure 6). The *hRluc*(wt)*-broccoli*-RNA produced by strain *C. glutamicum* ATCC 13032 (DE3) *Δcg2273* pSU1_P_{T7}-*hRluc*(wt)-*broccoli* revealed a 5.0-fold increased ratio of median fluorescence to cell size upon DFHBI addition, while the ratio of median fluorescence to cell size of the thermodynamically optimized *hRluc*(opt2)*-broccoli*-RNA was 7.2-fold increased (Figure 6). The results show that the abundance of RNA optimized for improved thermodynamic stability was significantly increased compared to the wildtype sequences.

### Example 6

eGFP protein expression encoded by a CDS that was optimized for improved RNA thermodynamic stability in *C. glutamicum* ATCC13032

### a) Construction of the plasmid vectors pSU3_P_{dapA-C6}_egfp(wt) and pSU3_P_{dapA-C6}_egfp(stabilized)

The construction of the plasmid vectors was achieved by means of chemical synthesis of synthetic DNA fragments. The DNA fragments include the promoter sequence P_{dapA-C6} (Vašicová, P., Pátek, M., Nešvera, J., Sahm, H., & Eikmanns, B. (1999). Analysis of the *Corynebacterium glutamicum* dapA promoter. *Journal of Bacteriology,* 181(19), 6188-6191. https://doi.org/10.1128/jb.181.19.6188-6191.1999), the wildtype CDS of *egfp* (*egfp* (wt); SEQ No.1) or the thermodynamically stabilized CDS of *egfp (egfp* (stabilized); SEQ ID No.35) (SEQ ID No.36 for *P*_{*dapA-C6*_}*egfp*(wt) and SEQ ID No.37 for P_{*dapA*-C6}_*egfp*(stabilized)). The thermodynamic stabilization of the egfp-RNA was conducted as described in example 1. The synthetic DNA fragments were incorporated in the vector pSU3 (SEQ ID No.38) by Gibson assembly resulting in vectors pSU3_P_{dapA-C6}_*egfp*(wt) (SEQ ID No.39) and pSU3_P_{dapA-C6}_*egfp*(stabilized) (SEQ ID No.40).

### b) Transformation of vectors pSU3, pSU3_P_{dapA-G6}_egfp(wt) and pSU3_P_{dapA-C6}_egfp(stabilized) in C. glutamicum ATCC13032

Competent cells of *C. glutamicum* strain ATCC13032 were prepared and transformed with plasmids pSU3, *P_{dapA-C6}*_*egfp*(wt) and P_{dapA-C6}_*egfp*(stabilized) according to Tauch et al. (Tauch, A., Kirchner, O., L6ffler, B., Götker, S., Pühler, A., & Kalinowski, J. (2002). Efficient electrotransformation of Corynebacterium diphtheriae with a mini-replicon derived from the Corynebacterium glutamicum plasmid pGA1. Current Microbiology, 45(5), 362-367. https://doi.org/10.1007/s00284-002-3728-3), respectively. The selection of the transformants was carried out on CGIII agar (1 %) plates with 25 µg/ml of kanamycin (Menkel, E., Thierbach, G., Eggeling, L., Sahm, H., & Asta Pharma Gruppe, D. A. (1989). Influence of Increased Aspartate Availability on Lysine Formation by a Recombinant Strain of Corynebacterium glutamicum and Utilization of Fumarate. In APPLIED AND ENVIRONMENTAL MICROBIOLOGY (Vol. 55, Issue 3).). Clones thus obtained were named *C. glutamicum* ATCC13032_pSU3, *C. glutamicum* ATCC13032_pSU3_P_{*dapA*-}*_{C6}_egfp*(wt) or *C. glutamicum* ATCC13032_pSU3_ P*_{dapA-C6}*_*egfp*(stabilized), depending on which plasmid was used for transformation.

### c) Cultivation and validation of cells using the BioLector system and fluorescence activated cell sorting (FACS)

The produced strains *C. glutamicum* ATCC13032_pSU3, *C. glutamicum* ATCC13032_pSU3_P*_{dapA-C6}_egfp*(wt) or *C. glutamicum* ATCC13032_pSU3_P_{*dapA*-C6}_*egfp*(stabilized) were each streaked on a BHI agar plate containing a final concentration of 25 µg/ml kanamycin and the plates were cultivated at 30°C for two days. Grown cells were inoculated in 10 mL BHI medium containing 25 µg/ml kanamycin and cultivated for 18 hours at 30°C by agitation at 250 rpm. Subsequently, grown cells were resuspended in fresh BHI medium containing 25 µg/ml kanamycin, and the OD₆₀₀ was adjusted to 0.2 in a 48-well microtiter Flowerplate (MFPs, Beckman Coulter GmbH, Baesweiler) containing 800 µL BHI medium with a final concentration of 25 µg/ml kanamycin. Cells were incubated at 30 °C and 1000 rpm in the BioLector cultivation system (Beckman Coulter GmbH, Baesweiler). After 24 hours of cultivation, cells were diluted to an OD₆₀₀ of 0.6 in PBS and the cell suspension was analyzed using an Arialll High-speed cell sorter (BD Biosciences, Franklin Lakes, NJ, USA) as described in example 1d.

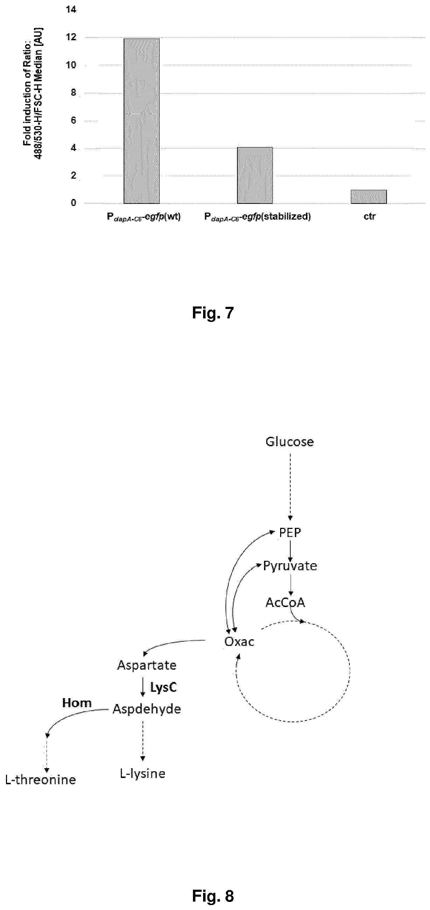

Compared to the empty vector control pSU3, cells containing vector pSU3_P_{dapA-}*_{C6}_egfp*(wt) showed a 12-fold increased fluorescent output (depicted as ratio of the median eGFP(488/530)-H to FSC-H) (Figure 7). In contrast, the cells containing vector pSU3_P_{dapA-C6}_*egfp*(stabilized) producing the eGFP protein from an RNA with a stabilized secondary structure featured only a four-fold increased fluorescent output compared to the empty vector control. The results show that RNA stabilization by improving the thermodynamic stability of RNA secondary structures significantly reduces protein expression and is efficient to modulate same in bacterial cells.

### Example 7

Increasing the lysine production of *C. glutamicum* by increasing expression of the key enzyme LysC and by reducing the expression of *horn* (key enzyme of a branching pathway) via RNA secondary structure modification to reroute the metabolic flux towards target molecule production

The feedback resistant variant of the lysine-sensitive aspartokinase encoded by *lysC*(fbr) constitutes a central enzyme in the metabolic pathway towards lysine production and is traditionally overexpressed using a strong promoter in lysine production strains (Figure 8) (Eggeling, L., & Bott, M. (2015). A giant market and a powerful metabolism: I-lysine provided by Corynebacterium glutamicum. In Applied Microbiology and Biotechnology (Vol. 99, Issue 8, pp. 3387-3394). Springer Verlag. https://doi.org/10.1007/s00253-015-6508-2). In contrast, the homoserine dehydrogenase encoded by *horn* is a key enzyme of the threonine production pathway branching off the lysine pathway (Figure 8). In lysine production strains, *horn* expression is usually reduced to increase the metabolic flux towards lysine production. The following example shows that the destabilization of RNA secondary structures leads to an increase of *lysC*(fbr) expression increasing lysine production, while the stabilization of RNA secondary structures of *horn* leads to a reduced translation of *horn* and hence, reduced by-product formation.

### a) Calculating a CDS with a destabilized RNA secondary structure of IysC(fbr) and a CDS with a stabilized RNA secondary structure of hom(wt)

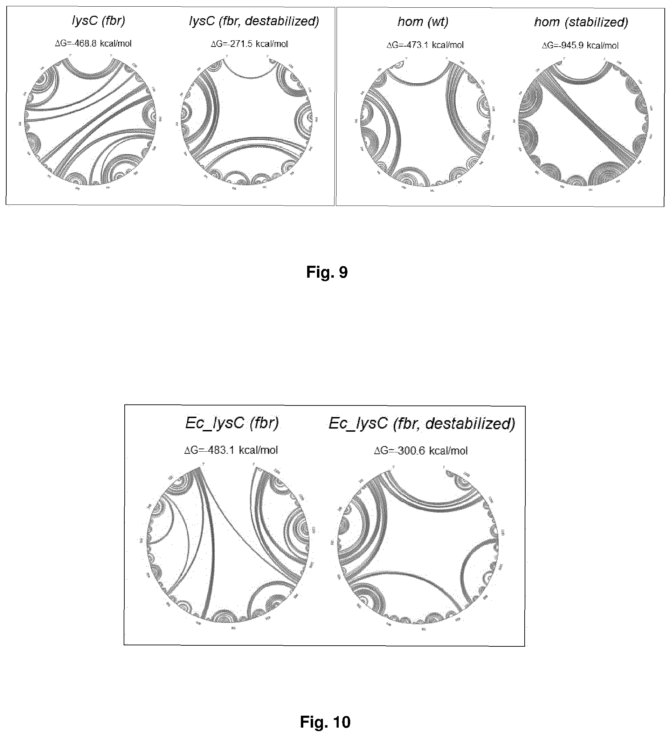

Using LinearFold, the MFE of the feedback resistant variant of *lysC* was determined to be ΔG= -468.8 kcal/mol (SEQ ID No.41) (Figure 9). A CDS of IysC(fbr) with a significantly reduced thermodynamically stability (MFE of ΔG=-271.5 kcal/mol determined by LinearFold) was determined and named /ysC(fbr,destabilized) (SEQ ID No.42). For the CDS of *hom*(wt)*,* a MFE of ΔG=-473.1 kcal/mol was determined by LinearFold (SEQ ID No.43). A CDS of *hom*(wt) resulting in an RNA secondary structure with a significantly increased thermodynamic stability (MFE of ΔG=-945.9 kcal/mol determined by LinearDesign) was calculated resulting in the sequence encoding hom(stabilized) (SEQ ID No.44) (Figure 9).

### b) Construction of the vectors pK19msB_lysC(fbr,destabilized) and pK19msB_hom(stabilized) for genomic integration in C. glutamicum

The construction of the plasmid vectors was achieved by means of chemical synthesis of synthetic DNA fragments. The DNA fragments include 500 bp upstream of the genomic integration site, the coding sequence of IysC(fbr, destabilized) or hom(stabilized) as calculated above, as well as 500 bp downstream of the genomic integration site. The DNA fragments were incorporated in the multiple cloning site of the integration vector pK19mobsacB (SEQ ID No.45) resulting in plasmids pK19msB_lysC(fbr,destabilized) (SEQ ID No.46) and pK19msB_hom(stabilized) (SEQ ID No.47).

### c) Genomic integration of /ysC(fbr,destabilized) and hom(stabilized) in C. glutamicum ATCC13032 lysC(fbr) to exchange chromosomally encoded CDS variants of lysC(fbr) and horn

Competent cells of the *C. glutamicum* strain ATCC13032 *lysC*(fbr) were prepared and transformed by electroporation with pK19msB_/ysC(fbr, destabilized) or pK19msB_*hom*(stabilized) according to Tauch et al. (Tauch, A., Kirchner, O., L6ffler, B., Götker, S., Pühler, A., & Kalinowski, J. (2002). Efficient electrotransformation of Corynebacterium diphtheriae with a mini-replicon derived from the Corynebacterium glutamicum plasmid pGA1. Current Microbiology, 45(5), 362-367. https://doi.org/10.1007/s00284-002-3728-3). The selection of the transformants was carried out on BHI (brain heart infusion) agar (1 %) plates with a final concentration of 25 µg/ml kanamycin. First and second recombination was conducted as previously described by Niebisch and Bott, 2001 (Niebisch, A., & Bott, M. (2001). Molecular analysis of the cytochrome bc1-aa3 branch of the Corynebacterium glutamicum respiratory chain containing an unusual diheme cytochrome c1. Archives of Microbiology, 175(4), 282-294. https://doi.org/10.1007/s002030100262). Resulting clones were verified by colony-PCR using primer 1 (SEQ ID No.48) and primer 2 (SEQ ID No.49) for integration in the genomically encoded *lysC*(fbr) region. The resulting fragment was digested using restriction enzyme *Xbal,* which only digests the *lysC*(fbr) but not the /ysC(fbr,destabilized) fragment. Using primer 3 (SEQ ID No.50) and primer 4 (SEQ ID No.51), the genomically encoded *horn* region was amplified. The fragment was tested for digestion with *Hind*III*,* and only clones with wildtype *horn* are digestible by this enzyme. The resulting strains are named *C. glutamicum lysC*(fbr, destabilized) and *C. glutamicum lysC*(fbr) hom(stabilized).

Additionally, competent cells of C. glutamicum lysC(fbr,destabilized) were transformed with plasmid pK19msB_hom(stabilized) and selection of positive integrants was done as described above. Resulting clones were verified by colony-PCR using primer 3 (SEQ ID No.50) and primer 4 (SEQ ID No.51) and the resulting fragment was tested for digestion with *Hind*III (only negative clones containing *hom*(wt) fragments are digestible). The resulting double mutant strain is named C. glutamicum *lysC*(fbr,destabilized)_*hom*(stabilized).

### d) Cultivation and HPLC validation of produced cells

The produced strains *C. glutamicum* /ysC(fbr,destabilized), *C. glutamicum* hom(stabilized), the double mutant *C. glutamicum lysC*(fbr,destabilized)_*hom*(stabilized) as well as the original strain *C. glutamicum* ATCC13032 *lysC*(fbr) were each streaked on a BHI agar plate and the plates were cultivated at 30°C overnight. As first pre-culture, one colony of grown cells was inoculated in a tube containing 2 mL BHI medium and incubated for eight hours at 120 rpm and 30°C. As second pre-culture, the first preculture was used to inoculate a baffled shake flask (100 mL total volume) containing 10 mL CGXII medium with 2% glucose. The flask was incubated at 120 rpm and 30°C for 17 hours. The next day, the second pre-culture was used to inoculate a baffled shake flask (500 mL total volume) to an OD₆₀₀ of 5 containing 50 ml CGXII medium with 2% glucose. The culture was incubated at 180 rpm and 30°C for 24 hours.

Subsequently, 1 mL cell culture was taken, centrifuged to 10 min at 13000 rpm and was diluted 1:100 in deionized water. Quantification of amino acids as ortho-phthaldialdehyde derivatives using ultra-high performance liquid chromatography (uHPLC) by automatic pre-column derivatization and separation by reverse-phase chromatography was done on an Agilent1290 Infinity LC ChemStation (Agilent, SantaClara, USA) equipped with a fluorescence detector. A gradient of Na-borate buffer (10 mM Na₂HPO₄; 10 mM Na₂B₄O₇, pH8.2; adapted to operator's guide) and methanol was used as the eluent for the Zorbax Eclipse AAA 3.5 µm 4.6×7.5 mm column (Agilent, SantaClara, USA).

HPLC results revealed a significantly increased amount of lysine produced by the strains expressing feedback-resistant and destabilized *lysC*(fbr, destabilized) compared to strains expressing the feedback-resistant, wildtype lysC(fbr). Moreover, strains expressing thermodynamically stabilized *horn* (hom(stabilized)) feature significantly reduced production of the by-product threonine. The double mutant encoding *lysC(*fbr, destabilized)_hom(stabilized) featured increased lysine and reduces threonine production. These results show that the stabilization and destabilization of RNA secondary structures provides an efficient strategy to fine-tune molecule production in *C. glutamicum.*

### Example 8

Increasing lysine production of *E. coli K- 12 MG1655* by increasing the expression of the key enzyme LysC by reducing RNA secondary structures

The feedback resistant variant of the lysine-sensitive aspartokinase III encoded by IysC(fbr) constitutes a central enzyme in the metabolic pathway towards lysine production and is traditionally overexpressed using a strong promoter in lysine production strains (Geng, F., Chen, Z., Zheng, P., Sun, J., & Zeng, A. P. (2013). Exploring the allosteric mechanism of dihydrodipicolinate synthase by reverse engineering of the allosteric inhibitor binding sites and its application for lysine production. Applied Microbiology and Biotechnology, 97(5), 1963-1971. https://doi.org/10.1007/s00253-012-4062-8 Kikuchi, Y., Kojima, H., & Tanaka, T. (1999). Mutational analysis of the feedback sites of lysine-sensitive aspartokinase of Escherichia coli. FEMS Microbiology Letters, 173(1), 211-215. https://doi.org/10.1111/j.1574-6968.1999.tb13504.x).

### a) Calculating a CDS with a stabilized RNA secondary structure of lysC(fbr)

Using LinearFold, the MFE of the feedback resistant *lysC* variant of *E*. *coli K-12 MG1655* was determined to be ΔG=-483.1 kcal/mol named Ec_*lysC*(fbr) (SEQ ID No.52) in the following (Figure 10). A thermodynamically destabilized CDS of *lysC(fbr)* with a MFE of ΔG=-300.6 kcal/mol was determined resulting in Ec_*lysC*(fbr,destabilized) (SEQ ID No.53).

### b) Design of the DNA fragments Ec_lysC(fbr)_FRT::neomycin and Ec_lysC(fbr,destabilized)_FRT::neomycin for replacing the native lysC copy in E. coli K-12 MG1655 via lambda RED recombination

The construction of the DNA fragments was achieved by means of chemical synthesis of the synthetic DNA fragments Ec*_lysC*(fbr)_FRT::*neomycin* (SEQ ID No.54) and Ec IysC(fbr,destabilized)_FRT::neomycin (SEQ ID No.55). The DNA fragments were flanked at the 5' and 3' end by each 60 nucleotides with homology to the target integration site. Additionally, the DNA fragments encode a neomycin phosphotransferase cassette flanked by FRT (FLP recognition target) sites for selection of positive recombinant clones.

### c) Genomic integration of Ec_lysC(fbr)_FRT::neomycin and Ec_lysC(fbr,destabilized)_FRT::neomycin in E. coli

Competent cells of the *E*. *coli* strain MG1655 pKD46 (SEQ ID No.56) were prepared and transformed by electroporation with the DNA fragments Ec_*lysC*(fbr)_FRT::*neomycin* and Ec IysC(fbr,destabilized)_FRT::neomycin according to the protocol of Datsenko and Wanner (Datsenko, K. A., & Wanner, B. L. (2000). One-step inactivation of chromosomal genes in Escherichia coli K-12 using PCR products. Proceedings of the National Academy of Sciences of the United States of America, 97(12), 6640-6645. https://doi.org/10.1073/pnas.120163297). The selection of the transformants was carried out on LB agar (1 %) plates with a final concentration of 50 µg/ml kanamycin. Resulting clones were verified by colony-PCR using primer 5 (SEQ ID No.57) and primer 6 (SEQ ID No.58) for replacing in the genomically encoded *lysC* region. Subsequently, the neomycin phosphotransferase cassette was removed by FLP recombinase activity encoded on plasmid pCP20 (SEQ ID No.59) as described by Datsenko and Wanner (Datsenko, K. A., & Wanner, B. L. (2000). One-step inactivation of chromosomal genes in Escherichia coli K-12 using PCR products. Proceedings of the National Academy of Sciences of the United States of America, 97(12), 6640-6645. https://doi.org/10.1073/pnas.120163297). Resulting strains were named *E. coli* IysC(fbr) and *E. coli* /ysC(fbr,destabilized).

### d) Cultivation and validation of produced cells by HPLC analysis

Strains *E*. *coli K- 12 MG1655, E. coli lysC*(fbr) and *E*. *coli* /ysC(fbr,destabilized) were each spread on LB agar (1%) plates and incubated over night at 37°C. Subsequently, a pre-culture containing 10 mL LB medium was inoculated with a colony picked off the agar plate and incubated for 17 hours at 37°C and 180 rpm. The next day, the main culture was inoculated to an OD₆₀₀ of 0.1 in a shaking flask containing 50 mL M9 minimal medium. The culture was incubated at 37°C and 180 rpm. After 24 hours, a sample was taken and measured by HPLC analysis as described in example 7.

For strains expressing the destabilized variant Ec_lysC(fbr,destabilized), a significantly increased amount of lysine was determined compared to the original strain expressing *lysC.* Additionally, strains expressing the feedback-resistant *lysC* variant (IysC(fbr)) featured increased lysine production compared to the original strain, but lower than the strain expressing Ec_lysC(fbr,destabilized).

### Example 9

Examination of thermodynamically optimized RNA secondary structures of *hRluc* produced in comparison to their corresponding wildtype sequences by the means of *in vitro* transcription using *C. glutamicum* crude extract

### a) Construction of the vectors encoding the wildtype and optimized CDS of hRluc

The modification of *hRluc*(wt) (SEQ ID No.3) resulting in *hRluc*(opt) (SEQ ID No.4) was performed are described in example 1. The construction of the vectors pSU1_P_{T7}-*hRluc*(wt) (SEQ ID No.62) and pSU1_P_{T7}-*hRluc*(opt) (SEQ ID No.63) was achieved by means of chemical synthesis of a synthetic DNA fragment and its incorporation into the vector pSU1 (SEQ ID No.9). The DNA fragments include the promoter sequence of P_{T7}, a 5'UTR, a CDS encoding *hRluc*(wt) or *hRluc*(opt) followed by a restriction site for enzyme *SnaB*I (SEQ ID No.60 for P_{T7}-*hRluc*(wt)-*SnaB*I and SEQ ID No.61 for P_{T7}*-hRluc*(opt)-*SnaB*I*).* Each DNA fragment was incorporated into the original vector pSU1 (SEQ ID No.9) resulting in plasmid vectors pSU1_P_{T7}-*hRluc*(wt) (SEQ ID No.61) and pSU1_P_{T7}-*hRluc*(opt) (SEQ ID No.63).

### b) Construction of the deletion vector pK19msB_Δcg2597

The construction of the plasmid vector was achieved by means of chemical synthesis of a synthetic DNA fragment and its incorporation into the vector pK19mobsacB. The 1615 bp DNA fragment 877 bp upstream of the *cg2597* coding site, the first 9 bp and final 12 bp of the *cg2597* open reading frame as well as 705 bp downstream of the *cg2597* coding site flanked by the restriction enzyme sites *BamH*I and *EcoR*I (SEQ ID No.64). The DNA fragment was digested by restriction enzymes *BamH*I and *EcoR*I*,* and the digested and purified fragment was ligated into the digested and purified vector pK19mobsacB (SEQ ID No.45) resulting in plasmid vector pK19msB_Δcg2597 (SEQ ID No.65).

### c) Genomic deletion of cg2597 in C. glutamicum ATCC13032 Δcg2273

Competent cells of the C. glutamicum ATCC13032 *Δcg2273* were prepared and transformed by electroporation with pK19msB _Δ*cg2597* according to Tauch et al. (Tauch, A., Kirchner, O., L6ffler, B., Götker, S., Pühler, A., & Kalinowski, J. (2002). Efficient electrotransformation of Corynebacterium diphtheriae with a mini-replicon derived from the Corynebacterium glutamicum plasmid pGA1. Current Microbiology, 45(5), 362-367. https://doi.org/10.1007/s00284-002-3728-3). The selection of the transformants was carried out on BHI (brain heart infusion) agar (1 %) plates with a final concentration of 25 µg/ml kanamycin. First and second recombination was conducted as previously described by Niebisch and Bott, 2001 (Niebisch, A., & Bott, M. (2001). Molecular analysis of the cytochrome bc1-aa3 branch of the Corynebacterium glutamicum respiratory chain containing an unusual diheme cytochrome c1. Archives of Microbiology, 175(4), 282-294. https://doi.org/10.1007/s002030100262). Resulting clones were verified by colony-PCR using primer 7 (SEQ ID No.66) and primer 8 (SEQ ID No.67) to verify the deletion of *cg2597.* The resulting strain is named *C. glutamicum* ATCC13032 Δ*cg2273 Δcg2597.*

### d) Construction of a vector encoding T7 polymerase

The construction of the plasmid vector was achieved by means of chemical synthesis of a synthetic DNA fragment (DE3; SEQ ID No.68) containing the *lacl* gene under control of the P*_{laclq}* promoter and *gene 1* of bacteriophage T7 under control of the P_{tac} promoter. The synthetic DNA fragment was incorporated into the pSU3 (SEQ ID No.38) plasmid, resulting in pSU3_DE3 (SEQ ID No.69).

### e) Transformation of C. glutamicum with pSU3_DE3

Competent cells of *C. glutamicum* strain ATCC13032 *Δcg2273 Δcg2597* were prepared and transformed with plasmid pSU3_DE3 according to Tauch et al (Tauch, A., Kirchner, O., Löffler, B., Götker, S., Pühler, A., & Kalinowski, J. (2002). Efficient electrotransformation of Corynebacterium diphtheriae with a mini-replicon derived from the Corynebacterium glutamicum plasmid pGA1. Current Microbiology, 45(5), 362-367. https://doi.org/10.1007/s00284-002-3728-3), resulting in strain *C. glutamicum* ATCC13032 *Δcg2273* Δ*cg2597_*pSU3_DE3.

### f) Cell extract preparation

A fresh colony of *C. glutamicum* strain ATCC13032 *Δcg2273* Δ*cg2597*_pSU3_DE3 was used to inoculate 5 mL CGIII medium with 25 µg/mL kanamycin in a test-tube. The culture was grown overnight at 30 °C by agitation at 160 rpm. The next day, the complete culture was used to inoculate 100 mL CGIII medium containing 25 µg/mL kanamycin in a 500 mL non-baffled shake flask to an OD₆₀₀ of 0.2. The flask was incubated at 30 °C by agitation at 200 rpm. After 12 hours of cultivation, induction was done using 1 mM IPTG. Cultivation was continued for further six hours. The cells were harvested using centrifugation and were washed twice with 10 mL ice-cold S30A buffer (14 mM magnesium-glutamate, 60 mM potassium-glutamate, 50 mM Tris(hydroxymethyl)aminomethane (TRIS), 2 mM Dithiothreitol, pH 7.7) per gram wet cells and once with S30 buffer (14 mM magnesium-glutamate, 60 mM potassium-glutamate, 10 mM TRIS, 2 mM Dithiothreitol, pH = 8.2) per gram wet cells. Pellets were flash-frozen and stored at -80 °C. Following, cell pellets were thawed and reconstituted in 1 mL S30 buffer per g cell pellet and homogenized to homogeneity using a Precellys Evolution homogenizer (Berkin instruments, Montigny-le-Bretonneux, France). The lysate was centrifuged for 10 min at 12,000 x rcf and 4 °C. Supernatant was flash-frozen and stored at -80 °C.

### g) In vitro transcription (IVT) using cell extract

Both plasmids (pSU1_P_{T7}-*hRluc*(wt) and pSU1_P_{T7}-*hRluc*(opt)) were digested with SnaBI to enable homogeneous transcription termination. 4 µg SnaBl-linearized pSU1_P_{T7}-*hRluc*(wt) or pSU1_P_{T7}-*hRluc*(opt) were mixed with adenosine triphosphate (ATP, 2 mM final concentration), cytosine triphosphate (CTP, 2 mM final concentration), guanidine triphosphate (GTP, 2 mM final concentration), uridine triphosphate (UTP, 2 mM final concentration) and 170 µL cell extract. Transcription reaction was performed at 37 °C for 80 min.

### h) Extraction of the RNA of interest and quantification

RNA was extracted from the IVT reactions using the Monarch total RNA kit (New England Biolabs, Ipswich, MA, USA). The isolated RNA was analyzed using an Agilent 5200 Fragment Analyzer (Agilent Technologies, Santa Clara, CA, USA).

The results revealed the production of RNA fragments of about 1000 nts, which corresponds to the expected size of RNA fragments *hRluc*(wt)-RNA and *hRluc*(opt)-RNA.

### Example 10

Examination of thermodynamically optimized RNA secondary structures of *hRluc* produced in comparison to their corresponding wildtype sequences by the means of *in vitro* transcription using *C. glutamicum* crude extract and nucleotides with modified nucleobases.

### a) Construction of the vectors encoding the wildtype and optimized CDS of hRluc

The construction of the plasmid vectors pSU1_P_{T7}-*hRluc*(wt) (SEQ ID No.61) and pSU1_P_{T7}-*hRluc*(opt) (SEQ ID No.63) is described in example 9.

### b) Cell extract preparation

The preparation of the cell extract was conducted as described in example 9.

### c) In vitro transcription (IVT) using cell extract

Both plasmids (pSU1_P_{T7}-*hRluc*(wt) and pSU1_P_{T7}-*hRluc*(opt)) were digested with SnaBI to enable homogeneous transcription termination. 4 µg SnaBl-linearized pSU1_P_{T7}-*hRluc*(wt) or pSU1_P_{T7}-*hRluc*(opt) were mixed with adenosine triphosphate (ATP, 2 mM final concentration), cytidine triphosphate (CTP, 2 mM final concentration), guanosine triphosphate (GTP, 2 mM final concentration), uridine triphosphate (UTP, 1 mM final concentration), Pseudouridine triphosphate (Pseudo-UTP, 1 mM final concentration) and 170 µL cell extract. Transcription reaction was performed at 37°C for 80 min.

### d) Extraction of the RNA of interest and quantification

RNA was extracted from the IVT reactions using the Monarch total RNA kit (New England Biolabs, Ipswich, MA, USA). The isolated RNA was analyzed using an Agilent 5200 Fragment Analyzer (Agilent Technologies, Santa Clara, CA, USA).

The results revealed the production of RNA fragments of about 1000 nts, which corresponds to the expected size of RNA fragments *hRluc*(wt)-RNA and hRluc(opt)-RNA.

### References

Zhang, H., Zhang, L., Lin, A., Xu, C., Li, Z., Liu, K., Liu, B., Ma, X., Zhao, F., Yao, W., Li, H., Mathews, D. H., Zhang, Y., & Huang, L. (2020). Algorithm for Optimized mRNA Design Improves Stability and Immunogenicity. http://arxiv.org/abs/2004.10177
Huang, L., Zhang, H., Deng, D., Zhao, K., Liu, K., Hendrix, D. A., & Mathews, D. H. (2019). LinearFold: Linear-time approximate RNA folding by 5'-to-3' dynamic programming and beam search. Bioinformatics, 35(14), i295-i304. https://doi.org/10.1093/bioinformatics/btz375
Terai, G., Kamegai, S., & Asai, K. (2016). CDSfold: an algorithm for designing a protein-coding sequence with the most stable secondary structure. Bioinformatics, 32(6), 828-834. https://doi.org/10.1093/bioinformatics/btv678
Eggeling, L., & Bott, M. (2015). A giant market and a powerful metabolism: I-lysine provided by Corynebacterium glutamicum. In Applied Microbiology and Biotechnology (Vol. 99, Issue 8, pp. 3387-3394). Springer Verlag. https://doi.org/10.1007/s00253-015-6508-2
Filonov, G. S., Moon, J. D., Svensen, N., & Jaffrey, S. R. (2014). Broccoli: Rapid selection of an RNA mimic of green fluorescent protein by fluorescence-based selection and directed evolution. Journal of the American Chemical Society, 136(46), 16299-16308. https://doi.org/10.1021/ja508478x
Geng, F., Chen, Z., Zheng, P., Sun, J., & Zeng, A. P. (2013). Exploring the allosteric mechanism of dihydrodipicolinate synthase by reverse engineering of the allosteric inhibitor binding sites and its application for lysine production. Applied Microbiology and Biotechnology, 97(5), 1963-1971. https://doi.org/10.1007/s00253-012-4062-8
Tauch, A., Kirchner, O., L6ffler, B., Götker, S., Pühler, A., & Kalinowski, J. (2002). Efficient electrotransformation of Corynebacterium diphtheriae with a mini-replicon derived from the Corynebacterium glutamicum plasmid pGA1. Current Microbiology, 45(5), 362-367. https://doi.org/10.1007/s00284-002-3728-3
Niebisch, A., & Bott, M. (2001). Molecular analysis of the cytochrome bc1-aa3 branch of the Corynebacterium glutamicum respiratory chain containing an unusual diheme cytochrome c1. Archives of Microbiology, 175(4), 282-294. https://doi.org/10.1007/s002030100262
Datsenko, K. A., & Wanner, B. L. (2000). One-step inactivation of chromosomal genes in Escherichia coli K-12 using PCR products. Proceedings of the National Academy of Sciences of the United States of America, 97(12), 6640-6645. https://doi.org/10.1073/pnas.120163297
Kikuchi, Y., Kojima, H., & Tanaka, T. (1999). Mutational analysis of the feedback sites of lysine-sensitive aspartokinase of Escherichia coli. FEMS Microbiology Letters, 173(1), 211-215. https://doi.org/10.1111/j.1574-6968.1999.tb13504.x
Vašicová, P., Pátek, M., Nešvera, J., Sahm, H., & Eikmanns, B. (1999). Analysis of the Corynebacterium glutamicum dapA promoter. *Journal of Bacteriology, 181*(19), 6188-6191. https://doi.org/10.1128/jb.181.19.6188-6191.1999
Chung, C. T., Niemela, S. L., & Miller, R. H. (1989). One-step preparation of competent Escherichia coli: transformation and storage of bacterial cells in the same solution. Proceedings of the National Academy of Sciences of the United States of America, 86(7), 2172-2175. https://doi.org/10.1073/pnas.86.7.2172
Menkel, E., Thierbach, G., Eggeling, L., Sahm, H., & Asta Pharma Gruppe, D. A. (1989). Influence of Increased Aspartate Availability on Lysine Formation by a Recombinant Strain of Corynebacterium glutamicum and Utilization of Fumarate. In APPLIED AND ENVIRONMENTAL MICROBIOLOGY (Vol. 55, Issue 3).

## Claims

1. A method for producing an RNA of interest, the method comprising
a) modifying the sequence of the RNA so that its secondary structure has a lower free energy than before the modification, and
b) introducing a DNA encoding the modified RNA of step a) into the cell.

2. The method according to claim 1, wherein the RNA encodes an amino acid sequence and is modified in step a) so that it still encodes the same amino acid sequence as before the modification.

3. The method according to claim 1 or 2, said method further comprising the steps of
• initiating transcription of the modified RNA; and/or
• extracting the modified RNA from the cell.

4. The method according to any of claims 1 to 3, wherein the amount of the RNA produced is increased compared to the same method in which the RNA without the modification is used.

5. The method according to any of claims 1 to 4, wherein the RNA is heterologous with respect to the cell in which it is produced.

6. A method for producing a cell showing an increased abundance of an RNA of interest in a cell, the method comprising
a) modifying the sequence of the RNA so that its secondary structure has a lower free energy than before the modification, and
b) introducing a DNA encoding the modified RNA of step a) into the cell.

7. A method for increasing the abundance of an RNA in a cell, the method comprising: introducing into the cell a DNA encoding the RNA, wherein the RNA is capable of forming a secondary structure, wherein the sequence of the RNA is modified so that its secondary structure has a lower free energy than before the modification.

8. A method for producing a protein of interest, the method comprising introducing into the cell a DNA encoding a modified RNA, wherein the RNA is capable of forming a secondary structure and encodes the protein of interest, wherein the sequence of the RNA is modified so that its secondary structure has a higher free energy than before the modification while still encoding the protein of interest.

9. A method for producing a molecule of interest in a cell, the method comprising introducing into the cell a DNA encoding a modified RNA, wherein the RNA is capable of forming a secondary structure and encodes a protein, wherein the sequence of the RNA is modified so that its secondary structure has a higher free energy than before the modification while still encoding the same amino acid sequence as before the modification and wherein the RNA or the protein encoded by the RNA is capable of upregulating the production of the molecule of interest.

10. A method for producing a molecule of interest in a cell, the method comprising introducing into the cell a DNA encoding a modified RNA, wherein the RNA is capable of forming a secondary structure and encodes an amino acid sequence, wherein the sequence of the RNA is modified so that its secondary structure has a lower free energy than before the modification while still encoding the same amino acid sequence as before the modification and wherein the RNA or the protein encoded by the RNA is capable of downregulating the production of the molecule of interest.

11. The method according to claim 9 or 10, wherein the amount of the molecule of interest that is produced is increased compared to the same method in which the RNA without the modification is used.

12. The method according to any of claims 1 to 11, wherein the cell is a bacterial cell, preferably from the genus *Corynebacterium* or *Escherichia.*

13. A cell comprising a modified RNA, wherein the RNA is capable of forming a secondary structure, wherein the sequence of the RNA is modified in that its secondary structure has a lower free energy than before the modification.

14. The cell according to claim 13, wherein the cell is from the genus *Corynebacterium* or *Escherichia.*

15. A pharmaceutical composition comprising an RNA, wherein the RNA is capable of forming a secondary structure and encodes an amino acid sequence, wherein the sequence of the RNA is modified so that its secondary structure has a lower free energy than before the modification while still encoding the same amino acid sequence as before the modification.
